# EUROPEAN PATENT SPECIFICATION

(11) **EP 4 566 661 B1**
(45) Date of publication and mention of the grant of the patent: **11.03.2026**
(21) Application number: 24177375.3
(22) Date of filing: 22.05.2024
(51) Int. Cl.: A61N 2/02

(54) **DEVICE FOR DEEP TRANSCRANIAL MAGNETIC STIMULATION REGULATION TREATMENT**
VORRICHTUNG ZUR BEHANDLUNG ZUR TIEFEN TRANSKRANIALEN MAGNETISCHEN STIMULATIONSREGULIERUNG
DISPOSITIF DE TRAITEMENT DE RÉGULATION DE STIMULATION MAGNÉTIQUE TRANSCRÂNIENNE PROFONDE

(30) Priority: 05.12.2023 CN 202311647965
(43) Date of publication of application: 11.06.2025
(73) Proprietor: Xuanwu Hospital, Capital Medical University, Xicheng District, Beijing 100053 (CN)
(72) Inventor: WANG, Yuping, Beijing, 100053 (CN)
(74) Representative: 2SPL Patentanwälte PartG mbB

(56) References cited:
- CN-A- 110 975 153
- CN-A- 114 028 720
- US-A1- 2017 014 637
- US-A1- 2019 046 794

## Description

### TECHNICAL FIELD

The present disclosure relates to the field of medical devices, and in particular to a device for deep transcranial magnetic stimulation regulation treatment.

### BACKGROUND

The technology for transcranial magnetic stimulation (TMS) is a noninvasive human brain stimulation technology, which can regulate the function of nerve tissues by providing repetitive pulsed magnetic fields. Numerous clinical studies have proved the effectiveness and safety of TMS in treating brain functional diseases. Existing magnetic stimulation modules include annular coils, "8"-shaped coils, biconical coils, and H-shaped coils, etc. However, the usually stimulation depth is 2-3 cm, and the deepest does not exceed 6 cm (H-shaped coils). Therefore, the existing magnetic stimulation methods cannot effectively reach the deep tissues of the human brain, such as temporal lobe, basal ganglia, thalamus, etc., but can only be used for the regulation and treatment of superficial sites of the brain, leading to the limitation on clinical application.

At present, the neural regulation technologies capable of reaching deep brain tissues such as temporal lobe, basal ganglia and thalamus are all invasive technologies, such as deep brain stimulation (DBS), which are expensive and incapable of adjusting target points once implanted. There is no noninvasive neural regulation technology capable of replacing DBS. Document US 2017 014637 A1 discloses systems comprising a plurality of coils and an electromagnetic field generator that energizes the plurality of coils so as to apply a tumor treating field to a specimen region.

CN110975153A discloses that: a magnetic field detection circuit is used to detect the magnetic field strength of the coil, and sends the detected signal to the main control board. And in the corresponding parts of the description, it is disclosed that: the magnetic field detection circuit is used to detect the magnetic field strength of the coil, to determine whether the magnetic field strength is within the safe range, and the magnetic field detection circuit sends the detected signal to the main control board. When the magnetic field strength exceeds the safe range, the main control board receives an abnormal signal from the field detection circuit and takes timely measures are taken.

US 2019/046794 A1 discloses that: the shutdown feature can be triggered responsive to one or more characteristics sensed by the sensor or another sensor that indicate a fault (e.g. a malfunction, defective component, unsafe operating condition, etc.); the wearable neural stimulation device can perform the self-test at least one of before treatment commences, during the treatment, or after the treatment ends; in an example, the self-test can include detecting a fault if the voltage at the low end of a power supply range is not within a preselected percentage (e.g.±5%) of a nominal range; it is noted that in some examples, the high end of the power supply range is not tested in order to avoid creating a large energy buildup that needs to be dissipated.

### SUMMARY

An objective of the present disclosure is to provide a device for deep transcranial magnetic stimulation regulation treatment, which makes a non-invasive transcranial magnetic field accurately reach a deep brain region by generating a superimposed electromagnetic field.

To achieve the objective above, the present disclosure employs the following technical solution.

A device for deep transcranial magnetic stimulation regulation treatment includes an upper computer control software, a system main control module, a stimulation coil excitation power module, and a deep stimulation coil module.

An output end of the upper computer control software is connected to an input end of the system main control module, an output end of the system main control module is connected to a control end of the stimulation coil excitation power module, and an output end of the stimulation coil excitation power module is connected to an input end of the deep stimulation coil module.

The upper computer control software is configured to send a stimulation instruction to the system main control module according to predetermined parameter data, where the predetermined parameter data includes a target region, excitation time, and an excitation current.

The system main control module is configured to send a control enable signal to the stimulation coil excitation power module when receiving the stimulation instruction.

The stimulation coil excitation power module is configured to provide a current to the deep stimulation coil module according to the control enable signal.

The deep stimulation coil module is configured to generate a superimposed magnetic field and transmit the superimposed magnetic field to the target region.

The deep stimulation coil module includes multiple coil groups. Each coil group includes two coils. There is an overlapping part among magnetic fields of the plurality of coil groups, and the overlapping part corresponds to a predetermined target region, and a current flowing through each coil group has same magnitude and direction, the plurality of coil groups are configured to be placed in different orientations with respect to a brain.

A position and an angle of each coil group are adjusted according to the target region, such that electromagnetic fields generated by respective coil groups are superimposed in the target region.

The device further comprises a power failure detection module; where the power failure detection module is connected to the system main control module and the stimulation coil excitation power module.

The power failure detection module is configured to collect an operating state of the stimulation coil excitation power module in real time, and to transmit the operating state to the system main control module; the system main control module is configured to control on/off of the stimulation coil excitation power module according to the operating state; the system main control module is further configured to transmit an off state of the stimulation coil excitation power module to the upper computer control software when controlling the stimulation coil excitation power module to be turned off; and the upper computer control software displays the off state of the stimulation coil excitation power module, and sends a stop instruction to the system main control module

According to the specific embodiments provided by the present disclosure, the present disclosure has the following technical effects: a superimposed electromagnetic field generated by controlling a deep stimulation coil module is transmitted to a target region, thus realizing that a non-invasive transcranial magnetic field accurately reaches a deep brain region.

### BRIEF DESCRIPTION OF THE DRAWINGS

To describe the technical solutions of the embodiments of the present disclosure or in the prior art more clearly, the following briefly introduces the accompanying drawings required for describing the embodiments. Apparently, the accompanying drawings in the following description show merely some embodiments of the present disclosure, and those of ordinarily skilled in the art may still derive other drawings from these accompanying drawings without creative efforts.
FIG. 1 is a structural diagram of a device for deep transcranial magnetic stimulation regulation treatment according to an embodiment of the present disclosure; and
FIG. 2 is a structural diagram of a stimulation module according to an embodiment of the present disclosure.

### DETAILED DESCRIPTION OF THE EMBODIMENTS

The following clearly and completely describes the technical solutions in the embodiments of the present disclosure with reference to the accompanying drawings in the embodiments of the present disclosure. Apparently, the described embodiments are merely a part rather than all of the embodiments of the present disclosure. All other embodiments obtained by a person of ordinarily skilled in the art based on the embodiments of the present disclosure without creative efforts shall fall within the protection scope of the present disclosure.

It is expected that the non-invasive transcranial magnetic field can accurately reaches intracranial cavity, so as to generate an induced current and biological effect in a target region without damaging nerve tissues in the region where the magnetic field passes. It is expected that multiple magnetic stimulation modules can focus on the same target point in the deep brain from different directions (similar to the principle of radiotherapy), and cumulative magnetic field on each path is far less than that of the focused target point, and has weak effect, and it is only at the target point that the induced current and magnetic field of physiological effect are generated. Through the registration of individualized brain magnetic resonance structural images, stimulation parameters before stimulation can be stimulated by designing the software, thus predicting a target-point position and physiological effect under the conditions of a stimulation magnetic field approach and stimulation parameters.

An objective of the present disclosure is to provide a device for deep transcranial magnetic stimulation regulation treatment, which makes a non-invasive transcranial magnetic field accurately reach a deep brain region by generating a superimposed electromagnetic field.

In order to make the objectives, technical solutions and advantages of the present disclosure more clearly, the present disclosure is further described in detail below with reference to the accompanying drawings and the embodiments.

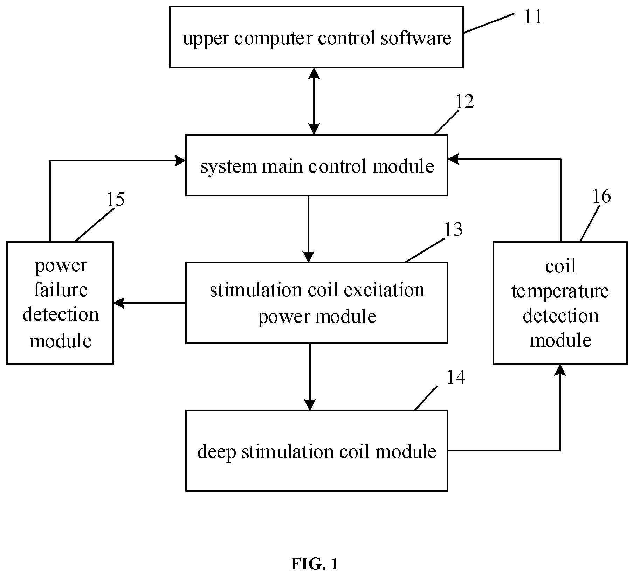

As shown in FIG. 1, a device for deep transcranial magnetic stimulation regulation treatment includes an upper computer control software 11, a system main control module 12, a stimulation coil excitation power module 13, and a deep stimulation coil module 14.

An output end of the upper computer control software 11 is connected to an input end of the system main control module 12, an output end of the system main control module 12 is connected to a control end of the stimulation coil excitation power module 13, and an output end of the stimulation coil excitation power module 13 is connected to an input end of the deep stimulation coil module 14.

The upper computer control software 11 is configured to send a stimulation instruction to the system main control module 12 according to predetermined parameter data. The predetermined parameter data includes a target region, excitation time, and an excitation current.

During specific implementation, the target region is obtained by a doctor through the examination of a patient, and the stimulation parameters such as excitation time and excitation current are obtained by the doctor according to the treatment regulation scheme, which is not the focus of the present disclosure, and thus will not be described in detail here.

The system main control module 12 is configured to send a control enable signal to the stimulation coil excitation power module 13 when receiving the stimulation instruction.

The stimulation coil excitation power module 13 is configured to provide a current to the deep stimulation coil module 14 according to the control enable signal.

The deep stimulation coil module 14 is configured to generate a superimposed magnetic field and transmit the superimposed magnetic field to the target region.

The deep stimulation coil module 14 includes multiple coil groups, and each coil group includes two coils. There is an overlapping part among magnetic fields of the multiple coil groups, and the overlapping part corresponds to a predetermined target region.

During specific implementation, a simulation experiment is performed according to an obtained position of the target region, so as to determine position data of each coil group. Each coil group is adjusted according to the position data of each coil group, there is an overlapping part between the magnetic fields generated by the multiple coil groups, and the overlapping part corresponds to an obtained position of the target region. After the adjustment of the multiple coil groups is completed, the target region is stimulated by the device for deep transcranial magnetic stimulation regulation treatment provided by the present disclosure. The position data includes three-dimensional coordinates and/or angular data.

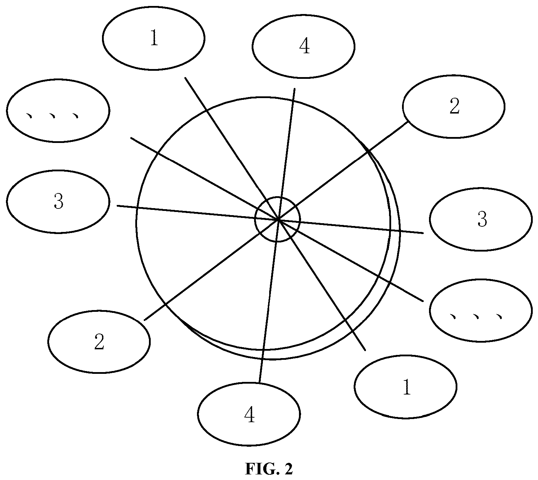

The same number in FIG. 2 denotes one coil group, a current flowing through each coil group has the same magnitude and direction. The multiple coil groups are placed in different orientations of the brain, and the superimposed electromagnetic field generated by each coil group in a certain target region of the brain is used to regulate and stimulate this region. The positions and angles of the coil groups can be adjusted according to different target regions (the target region is an existing established region in the medical field), so that the generated electromagnetic fields are superimposed in the target region, and the multiple coil groups are added for greater intensity of regulatory stimulation. The use of the multiple coil groups also reduces requirements on a coil excitation power supply, improves power usage efficiency, and reduces safety risks.

In some embodiments, the device for deep transcranial magnetic stimulation regulation treatment further includes a coil temperature detection module 16.

The coil temperature detection module 16 is connected to the system main control module 12, and the coil temperature detection module 16 is located inside the deep stimulation coil module 14.

The coil temperature detection module 16 is configured to collect a temperature of the deep stimulation coil module 14 in real time, and to transmit the temperature to the system main control module 12. The system main control module 12 is configured to control the on/ off of the stimulation coil excitation power module 13 according to the temperature. The system main control module 12 transmits the temperature to the upper computer control software 11. The upper computer control software 11 is configured to send a stop instruction to the system main control module 12 when the temperature is greater than a predetermined temperature threshold, and the upper computer control software 11 displays temperature failure information.

The deep stimulation coil module 14, after being powered on, generates an electromagnetic field required for regulation, so as to achieve the stimulation on a brain region. Simultaneously, the coil temperature detection module 16 is configured to detect the temperature of the deep stimulation coil module 14 in real time, and to send the temperature to the system main control module 12. The system main control module 12 can achieve the control (on/off) on the stimulation coil excitation power module 13 through hardware. The system main control module 12 further sends the temperature to the upper computer control software 11, and the upper computer control software 11 performs threshold comparison according to the temperature, and sends a stop instruction to the system main control module 12 when the temperature is greater than the predetermined temperature threshold. The upper computer control software 11 displays temperature failure information to prompt failure.

The device for deep transcranial magnetic stimulation regulation treatment further includes a power failure detection module 15.

The power failure detection module 15 is connected to the system main control module 12 and the stimulation coil excitation power module 13.

The power failure detection module 15 is configured to collect an operating state of the stimulation coil excitation power module 13 in real time, and to transmit the operating state to the system main control module 12. The system main control module 12 is configured to control the on/off of the stimulation coil excitation power module 13 according to the operating state. The system main control module 12 is also configured to transmit an off state of the stimulation coil excitation power module 13 to the upper computer control software 11 when controlling the stimulation coil excitation power module 13 to be turned off. The upper computer control software 11 displays the off state of the stimulation coil excitation power module 13, and sends a stop instruction to the system main control module 12.

The stimulation coil excitation power module 13 is configured to provide an operating current for the deep stimulation coil module 14 according to the signal sent by the system main control module 12. Simultaneously, the power failure detection module 15 is configured to detect an operating state of the stimulation coil excitation power module 13, and to send an abnormal state signal to the system main control module 12 when detecting the abnormal state signal (a power switching signal is detected, if the detected switching signal is 0, it is determined that the stimulation coil excitation power module 13 is in an abnormal state, and if the detected switching signal is 1, it is determined that the stimulation coil excitation power module 13 is in a normal state). The system main control module 12 is used to achieve the control (on/off) of the stimulation coil excitation power module 13 according to the signal sent by the power failure detection module 15.

According to the present disclosure, a non-invasive transcranial magnetic field can accurately reach a deep brain region by generating the superimposed electromagnetic field. The deep brain region is a brain region with a depth greater than 6 cm.

Various embodiments in this specification are described in a progressive way, and each embodiment focuses on the differences from other embodiments, so the same or similar parts in each embodiment can be referred to each other.

Specific examples are used herein for illustration of the principles and implementation methods of the present disclosure. The description of the embodiments is merely used to help illustrate the method and its core principles of the present disclosure. In addition, those of ordinarily skilled in the art can make various modifications in terms of specific embodiments and scope of application in accordance with the teachings of the present disclosure. In conclusion, the content of this specification shall not be construed as a limitation to the present disclosure.

## Claims

1. A device for deep transcranial magnetic stimulation regulation treatment, comprising an upper computer control software (11), a system main control module (12), a stimulation coil excitation power module (13), and a deep stimulation coil module (14);
an output end of the upper computer control software (11) is connected to an input end of the system main control module (12), an output end of the system main control module (12) is connected to a control end of the stimulation coil excitation power module (13), and an output end of the stimulation coil excitation power module (13) is connected to an input end of the deep stimulation coil module (14);
the upper computer control software (11) is configured to send a stimulation instruction to the system main control module (12) according to predetermined parameter data, wherein the predetermined parameter data comprises a target region, excitation time, and an excitation current;
the system main control module (12) is configured to send a control enable signal to the stimulation coil excitation power module (13) when receiving the stimulation instruction;
the stimulation coil excitation power module (13) is configured to provide a current to the deep stimulation coil module (14) according to the control enable signal;
the deep stimulation coil module (14) is configured to generate a superimposed magnetic field and transmit the superimposed magnetic field to the target region;
the deep stimulation coil module (14) comprises a plurality of coil groups; each coil group comprises two coils; there is an overlapping part among magnetic fields of the plurality of coil groups, the overlapping part corresponds to a predetermined target region, and a current flowing through each coil group has same magnitude and direction, the plurality of coil groups are configured to be placed on a head in different orientations during using;
a position and an angle of each coil group are adjusted according to the target region, such that electromagnetic fields generated by respective coil groups are superimposed in the target region; by generating a superimposed electromagnetic field, a non-invasive transcranial magnetic field is able to accurately reach a deep brain region, wherein the deep brain region is a brain region with a depth greater than 6 cm, and the plurality of coil groups are added for greater intensity of regulatory stimulation; a use of the plurality of coil groups also reduces requirements on a coil excitation power supply, improves power usage efficiency, and reduces safety risks
the device further comprises a power failure detection module (15);
**characterized in that**:
the power failure detection module (15) is connected to the system main control module (12) and the stimulation coil excitation power module (13);
the power failure detection module (15) is configured to collect an operating state of the stimulation coil excitation power module (13) in real time, and to transmit the operating state to the system main control module (12); the system main control module (12) is configured to control on/off of the stimulation coil excitation power module (13) according to the operating state; the system main control module (12) is further configured to transmit an off state of the stimulation coil excitation power module (13) to the upper computer control software (11) when controlling the stimulation coil excitation power module (13) to be turned off; and the upper computer control software (11) displays the off state of the stimulation coil excitation power module (13), and sends a stop instruction to the system main control module (12)

2. The device according to claim 1, further comprising a coil temperature detection module (16);
wherein the coil temperature detection module (16) is connected to the system main control module (12), and the coil temperature detection module (16) is located inside the deep stimulation coil module (14);
the coil temperature detection module (16) is configured to collect a temperature of the deep stimulation coil module (14) in real time, and to transmit the temperature to the system main control module (12); the system main control module (12) is configured to control on/off of the stimulation coil excitation power module (13) according to the temperature; the system main control module (12) transmits the temperature to the upper computer control software (11), and the upper computer control software (11) is configured to send a stop instruction to the system main control module (12) when the temperature is greater than a predetermined temperature threshold, and the upper computer control software (11) displays temperature failure information.

## Patentansprüche

1. Eine Vorrichtung zur Tiefe-Transkranielle-Magnetstimulations-Regulierungsbehandlung, umfassend eine obere Computersteuerungssoftware (11), ein Systemhauptsteuerungsmodul (12), ein Stimulationsspulenerregungsleistungsmodul (13) und ein Tiefe-Stimulations-Spulenmodul (14);
ein Ausgangsende der oberen Computersteuerungssoftware (11) ist mit einem Eingangsende des Systemhauptsteuerungsmoduls (12) verbunden, ein Ausgangsende des Systemhauptsteuerungsmoduls (12) ist mit einem Steuerungsende des Stimulationsspulenerregungsleistungsmoduls (13) verbunden, und ein Ausgangsende des Stimulationsspulenerregungsleistungsmoduls (13) ist mit einem Eingangsende des Tiefe-Stimulations-Spulenmoduls (14) verbunden;
die obere Computersteuerungssoftware (11) ist konfiguriert, um eine Stimulationsanweisung an das Systemhauptsteuerungsmodul (12) gemäß vorbestimmten Parameterdaten zu senden, wobei die vorbestimmten Parameterdaten eine Zielregion, eine Erregungszeit und einen Erregungsstrom umfassen;
das Systemhauptsteuerungsmodul (12) ist konfiguriert, um ein Steuerungsfreigabesignal an das Stimulationsspulenerregungsleistungsmodul (13) zu senden, wenn es die Stimulationsanweisung empfängt;
das Stimulationsspulenerregungsleistungsmodul (13) ist konfiguriert, um einen Strom an das Tiefe-Stimulations-Spulenmodul (14) gemäß dem Steuerungsfreigabesignal bereitzustellen;
das Tiefe-Stimulations-Spulenmodul (14) ist konfiguriert, um ein überlagertes Magnetfeld zu erzeugen und das überlagerte Magnetfeld an die Zielregion zu übertragen;
das Tiefe-Stimulations-Spulenmodul (14) umfasst eine Mehrzahl von Spulengruppen; jede Spulengruppe umfasst zwei Spulen; es gibt einen überlappenden Teil unter Magnetfeldern der Mehrzahl von Spulengruppen, der überlappende Teil entspricht einer vorbestimmten Zielregion, und ein Strom, der durch jede Spulengruppe fließt, hat die gleiche Größe und Richtung, die Mehrzahl von Spulengruppen sind konfiguriert, um während der Verwendung in verschiedenen Orientierungen auf einem Kopf platziert zu werden;
eine Position und ein Winkel jeder Spulengruppe werden gemäß der Zielregion angepasst, derart, dass elektromagnetische Felder, die durch jeweilige Spulengruppen erzeugt werden, in der Zielregion überlagert werden; durch Erzeugen eines überlagerten elektromagnetischen Feldes ist ein nicht-invasives transkranielles Magnetfeld in der Lage, eine tiefe Gehirnregion genau zu erreichen, wobei die tiefe Gehirnregion eine Gehirnregion mit einer Tiefe von mehr als 6 cm ist, und die Mehrzahl von Spulengruppen werden für eine größere Intensität der regulatorischen Stimulation hinzugefügt; eine Verwendung der Mehrzahl von Spulengruppen reduziert auch Anforderungen an eine Spulenerregungsleistungsversorgung, verbessert die Leistungsnutzungseffizienz und reduziert Sicherheitsrisiken;
die Vorrichtung umfasst ferner ein Leistungsausfalldetektionsmodul (15);
**dadurch gekennzeichnet, dass**:
das Leistungsausfalldetektionsmodul (15) mit dem Systemhauptsteuerungsmodul (12) und dem Stimulationsspulenerregungsleistungsmodul (13) verbunden ist;
das Leistungsausfalldetektionsmodul (15) konfiguriert ist, um einen Betriebszustand des Stimulationsspulenerregungsleistungsmoduls (13) in Echtzeit zu sammeln und den Betriebszustand an das Systemhauptsteuerungsmodul (12) zu übertragen; das Systemhauptsteuerungsmodul (12) konfiguriert ist, um ein Ein/Aus des Stimulationsspulenerregungsleistungsmoduls (13) gemäß dem Betriebszustand zu steuern; das Systemhauptsteuerungsmodul (12) ferner konfiguriert ist, um einen Aus-Zustand des Stimulationsspulenerregungsleistungsmoduls (13) an die obere Computersteuerungssoftware (11) zu übertragen, wenn das Stimulationsspulenerregungsleistungsmodul (13) gesteuert wird, um ausgeschaltet zu werden; und die obere Computersteuerungssoftware (11) den Aus-Zustand des Stimulationsspulenerregungsleistungsmoduls (13) anzeigt und eine Stoppanweisung an das Systemhauptsteuerungsmodul (12) sendet.

2. Die Vorrichtung gemäß Anspruch 1, ferner umfassend ein Spulentemperaturdetektionsmodul (16);
wobei das Spulentemperaturdetektionsmodul (16) mit dem Systemhauptsteuerungsmodul (12) verbunden ist und sich das Spulentemperaturdetektionsmodul (16) innerhalb des Tiefe-Stimulations-Spulenmoduls (14) befindet;
das Spulentemperaturdetektionsmodul (16) konfiguriert ist, um eine Temperatur des Tiefe-Stimulations-Spulenmoduls (14) in Echtzeit zu sammeln und die Temperatur an das Systemhauptsteuerungsmodul (12) zu übertragen; das Systemhauptsteuerungsmodul (12) konfiguriert ist, um ein Ein/Aus des Stimulationsspulenerregungsleistungsmoduls (13) gemäß der Temperatur zu steuern; das Systemhauptsteuerungsmodul (12) die Temperatur an die obere Computersteuerungssoftware (11) überträgt, und die obere Computersteuerungssoftware (11) konfiguriert ist, um eine Stoppanweisung an das Systemhauptsteuerungsmodul (12) zu senden, wenn die Temperatur größer als ein vorbestimmter Temperaturschwellenwert ist, und die obere Computersteuerungssoftware (11) zeigt Temperaturausfallinformationen an.

## Revendications

1. Dispositif pour un traitement de régulation de stimulation magnétique transcrânienne profonde, comprenant un logiciel de commande informatique supérieur (11), un module de commande principal de système (12), un module de puissance d'excitation de bobine de stimulation (13), et un module de bobine de stimulation profonde (14) ;
une extrémité de sortie du logiciel de commande informatique supérieur (11) est connectée à une extrémité d'entrée du module de commande principal de système (12), une extrémité de sortie du module de commande principal de système (12) est connectée à une extrémité de commande du module de puissance d'excitation de bobine de stimulation (13), et une extrémité de sortie du module de puissance d'excitation de bobine de stimulation (13) est connectée à une extrémité d'entrée du module de bobine de stimulation profonde (14) ;
le logiciel de commande informatique supérieur (11) est configuré pour envoyer une instruction de stimulation au module de commande principal de système (12) selon des données de paramètre prédéterminées, dans lequel les données de paramètre prédéterminées comprennent une région cible, un temps d'excitation, et un courant d'excitation ;
le module de commande principal de système (12) est configuré pour envoyer un signal d'activation de commande au module de puissance d'excitation de bobine de stimulation (13) lors de la réception de l'instruction de stimulation ;
le module de puissance d'excitation de bobine de stimulation (13) est configuré pour fournir un courant au module de bobine de stimulation profonde (14) selon le signal d'activation de commande ;
le module de bobine de stimulation profonde (14) est configuré pour générer un champ magnétique superposé et transmettre le champ magnétique superposé à la région cible ;
le module de bobine de stimulation profonde (14) comprend une pluralité de groupes de bobines ; chaque groupe de bobines comprend deux bobines ; il y a une partie de chevauchement parmi les champs magnétiques de la pluralité de groupes de bobines, la partie de chevauchement correspond à une région cible prédéterminée, et un courant circulant à travers chaque groupe de bobines a la même amplitude et la même direction, la pluralité de groupes de bobines sont configurés pour être placés sur une tête dans différentes orientations pendant l'utilisation ;
une position et un angle de chaque groupe de bobines sont ajustés selon la région cible, de sorte que les champs électromagnétiques générés par les groupes de bobines respectifs sont superposés dans la région cible ; en générant un champ électromagnétique superposé, un champ magnétique transcrânien non invasif est capable d'atteindre avec précision une région cérébrale profonde, dans lequel la région cérébrale profonde est une région cérébrale avec une profondeur supérieure à 6 cm, et la pluralité de groupes de bobines sont ajoutés pour une plus grande intensité de stimulation régulatrice ; une utilisation de la pluralité de groupes de bobines réduit également les exigences sur une alimentation de puissance d'excitation de bobine, améliore l'efficacité d'utilisation de puissance, et réduit les risques de sécurité ;
le dispositif comprend en outre un module de détection de panne de puissance (15) ;
**caractérisé en ce que** :
le module de détection de panne de puissance (15) est connecté au module de commande principal de système (12) et au module de puissance d'excitation de bobine de stimulation (13) ;
le module de détection de panne de puissance (15) est configuré pour collecter un état de fonctionnement du module de puissance d'excitation de bobine de stimulation (13) en temps réel, et pour transmettre l'état de fonctionnement au module de commande principal de système (12) ; le module de commande principal de système (12) est configuré pour commander l'activation/la désactivation du module de puissance d'excitation de bobine de stimulation (13) selon l'état de fonctionnement ; le module de commande principal de système (12) est en outre configuré pour transmettre un état désactivé du module de puissance d'excitation de bobine de stimulation (13) au logiciel de commande informatique supérieur (11) lors de la commande du module de puissance d'excitation de bobine de stimulation (13) pour qu'il soit désactivé ; et le logiciel de commande informatique supérieur (11) affiche l'état désactivé du module de puissance d'excitation de bobine de stimulation (13), et envoie une instruction d'arrêt au module de commande principal de système (12).

2. Dispositif selon la revendication 1, comprenant en outre un current flowing through each coil group has same magnitude and direction (16) ;
dans lequel le module de détection de température de bobine (16) est connecté au module de commande principal de système (12), et le module de détection de température de bobine (16) est situé à l'intérieur du module de bobine de stimulation profonde (14) ;
le module de détection de température de bobine (16) est configuré pour collecter une température du module de bobine de stimulation profonde (14) en temps réel, et pour transmettre la température au module de commande principal de système (12) ; le module de commande principal de système (12) est configuré pour commander l'activation/la désactivation du module de puissance d'excitation de bobine de stimulation (13) selon la température ; le module de commande principal de système (12) transmet la température au logiciel de commande informatique supérieur (11), et le logiciel de commande informatique supérieur (11) est configuré pour envoyer une instruction d'arrêt au module de commande principal de système (12) lorsque la température est supérieure à un seuil de température prédéterminé, et le logiciel de commande informatique supérieur (11) affiche des informations de panne de température.
